# EUROPEAN PATENT APPLICATION

(11) **EP 2 322 909 A1**
(43) Date of publication of application: **18.05.2011**
(21) Application number: 09450216.8
(22) Date of filing: 17.11.2009
(51) Int. Cl.: G01N 3/38, A61F 2/82

(54) **Apparatus, components and methods for fatigue testing of stents**

(71) Applicant: Universität Wien, 1010 Wien (AT)
(72) Inventor: Weiss, Brigitte, 1180 Wien (AT); Khatibi, Golta, 1030 Wien (AT)
(74) Representative: Weiser, Andreas

(57) **Abstract**

The invention relates to an apparatus (5) for the fatigue testing of stents, comprising a solid body (6) with at least one bore (15) for receiving a stent (1) with tight fit and means (7) for generating a standing sonic wave (8) within the solid body (6). The invention further relates to components and methods for the fatigue testing of stents.

## Description

### Background of the Invention

The present invention relates to an apparatus, a component and a method for the fatigue testing of stents.

Stents are tubular medical implants for insertion into a passage or conduit of the body to support the wall thereof from the inside. Stents are used in a wide variety of applications such as coronary, urethral or vascular surgery. In most cases stents are expandable mesh tubes which are inserted into the body passage in a contracted state threaded on a balloon catheter, the latter then being inflated to expand the stent to its tubular form which supports the wall of the passage. Stents come in many different types and forms, made from various materials including metals and synthetics, are usual laser-cut to create an expandable mesh-like structure therein, can be impregnated with pharmaceutical compositions ("drug eluting stents", DES), can be covered or cladded with fabrics ("graft stents", "grafts"), et cet., all of which are comprised by the term "stent" used herein.

To meet clinical and regulatory requirements for their long-term in-vivo-durability, e.g. as defined in standard DIN EN 12006-3, stents must undergo severe fatigue strength tests before market approval. It should be noted that a stent in e.g. a coronary artery is expected to endure millions of load cycles during its lifetime due to the oscillating diameter changes of the artery following the blood pressure pulse. Test facilities for stents should therefore be able to generate cyclic strains with a maximum of load cycles and in a test bed as realistic as possible.

To this end, hitherto stents have been tested by inserting them into a flexible synthetic hose which is mounted between the counteracting pistons of a conventional mechanical deflection strain tester subjecting the hose - and the stent therein - to cyclic deflections while passing a blood-like liquid through the hose. Another possibility are hydraulic strain testers which create cyclic pressure changes within the hose to deform the stent. Testing devices of these kinds can generate load cycles in the frequency range of 100 to 300 Hz due to their mechanical restrictions. Thus, the number of load cycles which can be achieved within a reasonable time span is very limited.

### Object of the Invention

It is an object of the invention to provide an apparatus, a component and a method for the fatigue testing of stents which can impose an increased number of load cycles on a stent. It is a further object of the invention to devise such methods, components and apparatus which provide for a realistic in-vivo-experience of the test environment for the stent, in particular with less complex means than in the state of the art.

### Summary of the Invention

In a first aspect of the invention these objects are met with an apparatus for the fatigue testing of stents, comprising a solid body with at least one bore for receiving a stent with tight fit and means for generating a standing sonic wave within the solid body.

The invention is based on the insight that a bore in a solid body which resonates in a standing wave is subjected to cyclic form changes, due to the mass movements within the body, which can be directly imposed on a stent expanded within the bore. In this way a much higher number of load cycles can be achieved as compared to the piston or hydraulic devices of the state of the art.

Preferably, the generating means generate an ultrasonic wave. With this embodiment e.g. up to 50.000 load cycles per second can be generated, resulting in test runs of 10⁸ to 10¹⁰ load cycles within a reasonable amount of test time.

According to a preferred embodiment of the invention the bore is oblique, preferably transverse, to the direction of the standing wave. Thus, the stent will be subjected to changing radial deformations as will be detailed later on.

Preferably, the bore is situated at a wave node of the standing wave in order to achieve maximum efficiency in the form changes of the bore.

According to a further preferred embodiment of the invention the solid body is coupled to the generating means at one end only and has a length equal to an odd multiple of λ/2 of the standing wave. Leaving the other end of the solid body free to oscillate obviates the need for complex tuning measures.

The generating means preferably comprise a sonic source and a horn for coupling the sonic source to the solid body in order to increase the amplitude of the mass movements within the body.

In one embodiment, the solid body comprises at least two bars crossing each other in the same plane, wherein at least one bore is in the center of the crossing. Alternatively, the solid body can be in the form of a disc, at least one bore being in the center of the disc.

In all embodiments, the apparatus can additionally comprise means for passing a fluid through the stent. The fluid can be heated to provide a realistic in-vivo scenario for the stent while being tested.

The bore can have any cross-section similar to any passage or conduit of a living body. For the regular case of tubular stents the bore is preferably of circular cross-section.

The bore can have a curved axis to impose non-radial strains onto the stent and/or to receive stents which follow a curve when implanted. Furthermore, the bore can even be branched in order to receive branched stents.

In a second aspect of the invention, there is provided a component for the above disclosed apparatus, comprising a solid sonic resonator having at least one bore for receiving a stent. The resonator resonates preferably in at least one direction transverse to the bore.

In a third aspect the objects of the invention are achieved with a method for the fatigue testing of stents, comprising the steps of:
inserting a stent into a bore of a solid body,
expanding the stent to tightly fit into the bore, and
resonating the solid body with a standing wave.

Preferably, the standing wave is an ultrasonic wave, and in particular the bore is located at a wave node of the standing wave.

The solid body is preferably resonated in at least one direction transverse to the bore.

Further variants of the method comprise the step of passing a fluid through the stent while resonating the solid body, and in particular the step of heating the fluid.

### Short Description of the Drawings

The invention will be further described under reference to the enclosed drawings in which:
Fig. 1 shows an expandable stent according to the state of the art, partially deployed from its delivery system, in a side view;
Fig. 2 is a side view of an apparatus of the invention for testing the fatigue strength of stents;
Fig. 3 is a diagram of sonic waves propagating in the apparatus of Fig. 2;
Figs. 4a and 4b are elevational and side views, respectively, of the resonator of the apparatus of Fig. 2;
Figs. 5a and 5b are enlarged detailed elevational views of the bore of the resonator of Figs. 4a and 4b in two different states of deformation during the resonation of the resonator;
Figs. 6a - 6d show different embodiments of the resonator of the invention in one elevational and three sectional views, respectively; and
Figs. 7a - 7c represent further embodiments of the resonator of the invention in three elevational views.

### Detailed Description of the Preferred Embodiments

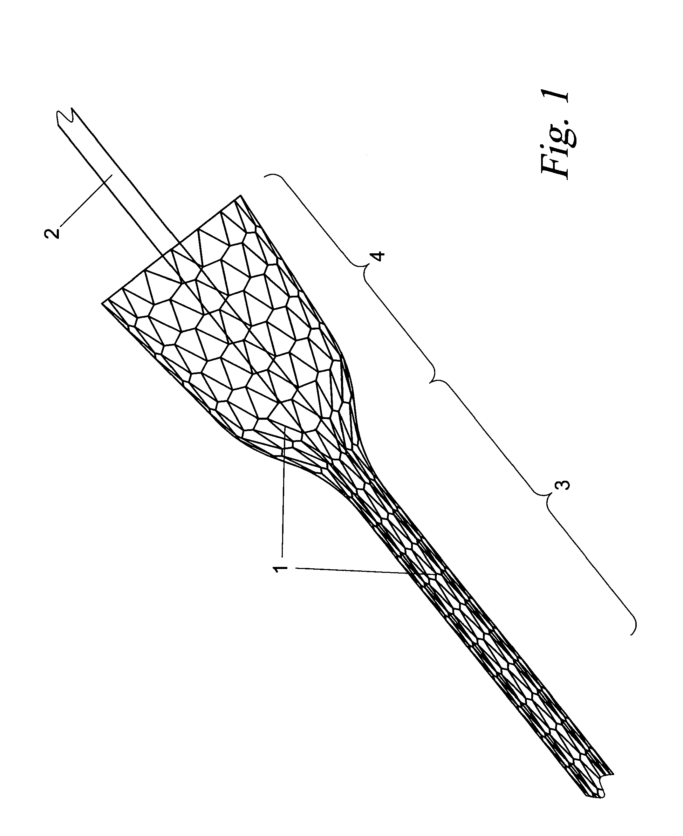

Fig. 1 shows a stent 1 threaded on an inflatable filament-like balloon catheter 2 and partially deployed for purposes of illustration, i.e. a first section 3 of the stent 1 being shown in its not-expanded state and a second section 4 of the stent 1 being shown in its expanded state. As is known to the man skilled in the art, in implantation surgery stent 1 is delivered to its application site and expanded there by means of the catheter 2, which is then deflated and withdrawn. The stent 1 can be made from any suitable material, e.g. a metal or synthetic mesh which has usually been laser-cut from a tube. The stent 1 can be provided with pharmaceutical drugs, covered and/or cladded with fabrics or tissues, et cet.

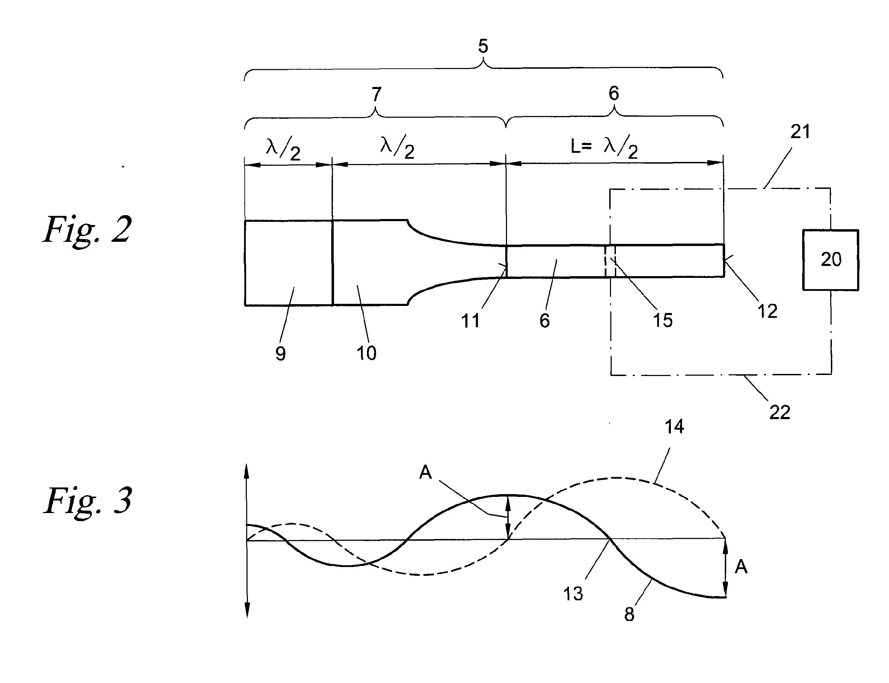

Fig. 2 shows an apparatus 5 for the fatigue testing of stents such as the stent 1 of Fig. 1. Apparatus 5 comprises a sonic resonator 6 in the form of a solid body which is coupled to a generating means 7 for generating a standing sonic wave 8 (Fig. 3) within the resonator 6. The resonator 6 can be manufactured from any solid material, e.g. metals, synthetics, ceramics et cet. The generating means 7 can be any suitable device for generating and coupling a sonic wave into the resonator 6, i.e. any means for inducing the resonator 6 to resonate in a standing wave mode. For example, generating means 7 can be comprised of a sonic source 9, e.g. a piezo-electric transducer, followed by a tapered horn 10 which increases the amplitude A of the sonic wave 8 before it is coupled into the resonator 6.

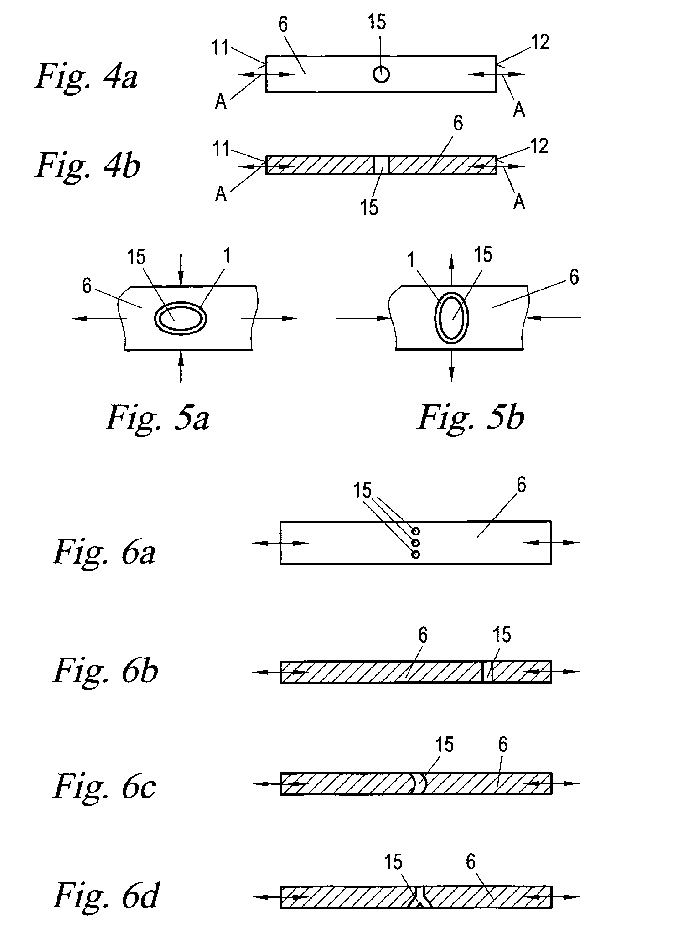

According to Figs. 2, 4a and 4b the resonator 6 is - at least partially - a solid body, here in form of an elongated bar having a length L equal to an odd multiple of the half-wavelength (λ/2) of the standing wave 8 resonating therein, coupled at its one end 11 to the generating means 7 and free at its other end 12.

In this way, the resonator 6 resonates primarily in a longitudinal mode and its ends 11, 12 move reciprocally and opposite to one another, each with an amplitude A. Wave node 13 of the stationary wave 8 - as regards this longitudinal resonation or length oscillation, respectively - is therefore in the middle of the resonator length L. In terms of the internal expansion and compression forces created within the material of the solid body of the resonator 6, which forces are shown in Fig. 3 by broken-line curve 14, these forces have their maximum at the wave node 13 and their minima at the ends 11, 12.

It is at this very wave node 13, i.e. the point of maximal compression and expansion forces exerted on the material of the solid body 6, that a bore (hole) 15 is created through the solid body sonic resonator 6. The bore 15 receives the stent 1 to be tested (the sample) with tight fit.

That means, for performing fatigue tests on a stent 1 the stent 1 is introduced into the bore 15 of the solid body sonic resonator 6 and then expanded, e.g. by a conventional delivery system such as the catheter 2, to its regular form of use in which it tightly contacts the inner wall of the bore 15.

In use of the apparatus 5, when the resonator 6 is induced to resonation by the generating means 7, the bore 15 thus experiences a cyclic deformation which can be seen in cross-section, or elevation, respectively, in Figs. 5a and 5b (which show the bore 15 in an exaggerated size and deformation for purposes of illustration): If the bore 15 has e.g. a circular cross-section in its state of rest, then the cross-section cycles in use between two oval forms the main axes of which are perpendicular to one each other. In this way, the stent 1 which is tightly received in the bore 15 is subjected to the same cyclic cross-oval deformation, which imposes corresponding cyclic radial strains on the tubular wall or mesh, respectively, of the stent 1.

The bore 15 is preferably transverse to the direction of the standing wave 8 within the resonator 6. In the case of a bar-like resonator 6 resonating longitudinally the bore 15 is thus preferably transverse to the longitudinal direction of the bar. However, other directions of the bore 15 could also be contemplated, e.g. any oblique direction with respect to the standing wave 8 in order to perform specialized tests on a stent.

If the generating means 7 generates a sonic wave with a high frequency, e.g. above 5.000 Hz, and preferably in the ultrasonic range, particularly preferred above 20 kHz, most preferred between 20 and 50 kHz, then 10⁹ - 10¹⁰ load cycles can be achieved within a testing time of only one day.

After the testing time the stent 1 has spent in the apparatus 5 the stent 1 is removed from the apparatus 5 and its fatigue damage can be examined by conventional means, e.g. by inspection with an endoscope, an optical or a scanning electron microscope. Such examinations can even be made when the stent 1 is still within the bore 15.

Fig. 6a shows an embodiment of the resonator 6 which houses more than one bore 15, e.g. several parallel bores 15 lying within the plane of the wave node 13.

According to Fig. 6b the bore(s) 15 could also be situated at locations different from the wave node plane 13, i.e. intermediate between the wave node 13 and one of the ends 11, 12. In this way, additional axial strains on the stent 1 could be generated.

Fig. 6c shows an embodiment with a bore 15 which follows a curved axis. This can be used on the one hand for stents 1 having a curved axis, or on the other hand for introducing additional non-radial strains on a straight stent 1 fit into the bore 15.

According to the embodiment of Fig. 6d the bore 15 can be specifically adapted to house irregularly formed stents 1, e.g. branched stents.

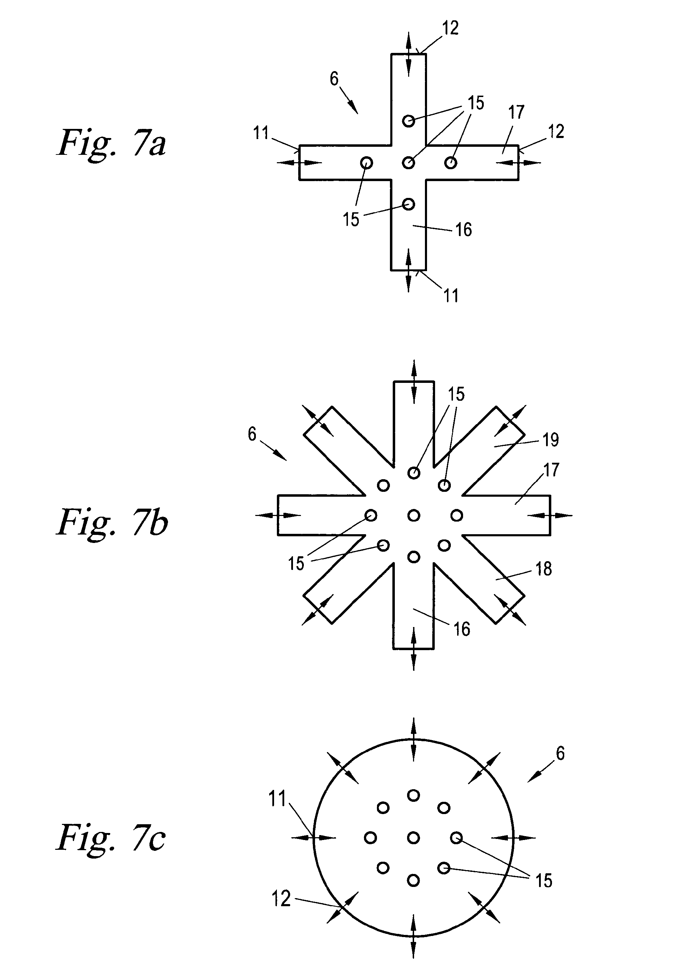

The resonator 6 can take other forms than the longitudinal or bar embodiment of Figs. 2 - 6, as will be shown with reference to Figs. 7a - 7c. For example, two crossing bars 16, 17 could be used, each resonating longitudinally, i.e. with swinging ends 11, 12. The wave node resides in the center of the cross where preferably at least one bore 15 is located. Further bores 15 can be provided elsewhere within the solid body of the resonator 6.

In the embodiment of Fig. 7a it is e.g. sufficient to couple the sonic wave into only one of the four ends 11, 12, since the other three ends will resonate freely. Optionally, each bar 16, 17 could be coupled to a separate generating means 7 generating sonic waves with a varying phase offset or a slight frequency offset in order to produce cyclic strains with rotating direction, e.g. oval oscillations according to Figs. 5a and 5b which slowly rotate in their direction around the axis of the bore 15. In this way complex test pattern cycles could be realized.

Fig. 7b shows another embodiment with four bars 16, 17, 18 and 19 crossing in one plane and a multitude of bores 15 circularly disposed around a central bore 15. Again, two or more of the bars 16 - 19 could each be provided with a separate generating means 7 in order to induce modulated standing wave modes in the bars and complex modulated form changes in the bores 15.

Fig. 7c shows a further generalization of the concept of Fig. 7b wherein the resonator 6 is in the form of a disc which is induced to various disc resonant modes, as they are known in the art, by suitable excitation with multiple generating means 7 at peripheral points 11, 12 et cet. of the disc.

In all embodiments the apparatus 5 can be provided with means 20 (Fig. 1) for passing a fluid, e.g. a liquid resembling the characteristics of blood, through the stent 1 in the bore 5. The means 20 can e.g. be a pump circulating the fluid via flexible hoses 21, 22 connected to the openings of the bore 15 through the stent 1. The means 20 can additionally heat the fluid up to a physiological temperature of e.g. 37°C in order to create a test bed environment for the stent 1 as realistic as possible to an in-vivo use.

In general the bore 15 need not be a through-bore passing through the resonator 6 but could also be a "blind hole" closed at one side. A through-bore is, however, preferred in that it creates more consistent deformations of the bore wall. The resonator 6 need not necessarily be made entirely of solid material but could also have recesses, cavities et cet., e.g. for controlling the propagation of the standing wave therein. The resonator 6 could also be excited on both ends, if it is in the form of a bar, or on more than one point, if it has a more general form. Sonic generators for multiple excitation should preferably be tuned, or matched, respectively, in order to generate a resonance in the resonator 6, be it stationary or modulated as exemplified above.

The invention is thus not restricted to the specific embodiments disclosed herein but encompasses all modifications, combinations and variations thereof which fall into the scope of the appended claims.

## Claims

1. An apparatus for the fatigue testing of stents, comprising a solid body (6) with at least one bore (15) for receiving a stent (1) with tight fit and means (7) for generating a standing sonic wave (8) within the solid body (6).

2. The apparatus of claim 1, wherein the generating means (7) generate an ultrasonic wave (8).

3. The apparatus of claim 1 or 2, wherein the bore (15) is oblique, preferably transverse, to the direction of the standing wave (8).

4. The apparatus of any of the claims 1 to 3, wherein the bore (15) is situated at a wave node (13) of the standing wave (8).

5. The apparatus of any of the claims 1 to 4, wherein the solid body (6) is coupled to the generating means (7) at one end only and has a length (L) equal to an odd multiple of λ/2 of the standing wave (8).

6. The apparatus of any of the claims 1 to 5, wherein the generating means (7) comprise a sonic source (9) and a horn (10) for coupling the sonic source (9) to the solid body (6).

7. The apparatus of any of the claims 1 to 6, wherein the solid body (6) comprises at least two bars (16 - 19) crossing each other in the same plane, at least one bore (15) being in the center of the crossing.

8. The apparatus of any of the claims 1 to 6, wherein the solid body (6) is in the form of a disc, at least one bore (15) being in the center of the disc.

9. The apparatus of any of the claims 1 to 8, comprising means (20) for passing a fluid through the bore (15).

10. The apparatus of any of the claims 1 to 9, wherein the bore (15) is of circular cross-section.

11. The apparatus of any of the claims 1 to 10, wherein the bore (15) has a curved axis.

12. The apparatus of any of the claims 1 to 11, wherein the bore (15) is branched.

13. A component for the fatigue testing of stents, comprising a solid sonic resonator (6) having at least one bore (15) for receiving a stent (1).

14. The component of claim 13, wherein the resonator (6) resonates in at least one direction transverse to the bore (15).

15. A method for the fatigue testing of stents, comprising the steps of:
inserting a stent (1) into a bore (15) of a solid body (6),
expanding the stent (1) to tightly fit into the bore (15), and
resonating the solid body (6) with a standing wave (8).

16. The method of claim 15, wherein the standing wave (8) is an ultrasonic wave.

17. The method of claim 15 or 16, wherein the bore (15) is located at a wave node (13) of the standing wave (8).

18. The method of any of the claims 15 to 17, wherein the solid body (6) is resonated in at least one direction transverse to the bore (15).

19. The method of any of the claims 15 to 18, comprising the step of passing a fluid through the stent (1) while resonating the solid body (6).

20. The method of claim 19, comprising the step of heating the fluid.
